# EUROPÄISCHE PATENTSCHRIFT

(11) **EP 1 348 409 B1**
(45) Veröffentlichungstag und Bekanntmachung des Hinweises auf die Patenterteilung: **29.12.2004**
(21) Anmeldenummer: 03002204.0
(22) Anmeldetag: 31.01.2003
(51) Int. Cl.: A61F 2/64

(54) **Prothesen-Kniegelenk mit einem hydraulischen Dämpfungszylinder**
Prosthetic knee joint with hydraulic shock absorber
Prothese d'articulation du genou avec amortisseur hydraulique

(30) Priorität: 28.03.2002 DE 10214357
(43) Veröffentlichungstag der Anmeldung: 01.10.2003
(73) Patentinhaber: Otto Bock HealthCare GmbH, 37115 Duderstadt (DE)
(72) Erfinder: Mosler, Lüder, Dr., 37115 Duderstadt (DE)
(74) Vertreter: Lins, Edgar, Dipl.-Phys. Dr.jur.

(56) Entgegenhaltungen:
- DE-A- 19 859 931
- GB-A- 2 111 171
- GB-A- 2 244 006

## Beschreibung

Die Erfindung betrifft ein Prothesen-Kniegelenk mit einem hydraulischen Dämpfungszylinder gemäß dem Oberbegriff des Anspruches 1, wie z.B. aus DE-19859931-A bekannt. Die Dämpungseigenschaften eines Hydraulikzylinders für den Einsatz in einem prothetischen Knie kann steuerbar ausgeführt sein, um den unterschiedlichen Widerstandsanforderungen in der Schwung- und Standphase gerecht zu werden. Eine Möglichkeit zur Anpassung besteht in einer elektronischen Steuerung über elektrische Stellglieder, die entweder die Strömungsbedingungen verändern oder die viskosen Eigenschaften der Flüssigkeit beeinflussen. Eine Veränderung der Strömungsbedingungen kann über ein Stellventil, die der viskosen Eigenschaften mittels Beeinflussung z.B. magnetorheologischer Flüssigkeiten vorgenommen werden.

Bei der Veränderung viskoser Eigenschaften einer Flüssigkeit ist aus dem Stand der Technik bekannt, dass ein Stellglied in Abhängigkeit von der Bewegungsphase die Viskosität der Flüssigkeit durch Anlegen eines Magnetfeldes verändert, wobei ein hoher Widerstand und damit eine hohe Viskosität insbesondere in der Standphase erforderlich ist. Da die Standphase in der Regel länger als die Schwungphase dauert, wird ständig ein relativ hoher Strom benötigt, um ein Stehen sicher zu ermöglichen.

Ausgehend von diesem Stand der Technik liegt der Erfindung die Aufgabe zugrunde, ein Prothesen-Kniegelenk bereitzustellen, das mit einem niedrigeren Stromverbrauch gesteuert werden kann und zudem eine erhöhte Sicherheit beim Ausfall der Steuerung gewährleistet.

Erfindungsgemäß wird diese Aufgabe durch ein Prothesen-Kniegelenk mit den Merkmalen des Anspruchs 1 gelöst.

Vorteilhafte Ausgestaltungen und Weiterbildungen der Erfindung sind in den Unteransprüchen dargestellt.

Durch ein ein permanentes Kraftfeld erzeugendes Stellglied, das die Hydraulikflüssigkeit mit einem bestehenden Kraftfeld beaufschlagt, das durch das elektronisch gesteuerte Kraftfeld geschwächt oder gestärkt wird, wird erreicht, dass für die Schwungphase nur ein geringer Strom angelegt werden muss, um das bestehende Kraftfeld durch das elektronisch gesteuerte Kraftfeld zu schwächen oder aber die Standsicherheit durch ein entsprechend anders orientiertes, elektronisch gesteuertes Kraftfeld zu verstärken. Bei einem Ausfall der elektronischen Steuerung wird so weiterhin die Standsicherheit des Prothesenträgers gewährleistet und eine, wenn auch eingeschränkte Bewegungsmöglichkeit bleibt bestehen. Darüber hinaus können die Wartungsintervalle des Prothesen-Kniegelenkes verlängert werden, da ein insgesamt geringerer Stromverbrauch auftritt.

Eine Ausgestaltung der Erfindung sieht ein vormagnetisiertes Stellglied vor, das eine magnetorheologische Hydrauligflüssigkeit mit einem Magnetfeld beaufschlagt.

Vorteilhafterweise weist das Stellglied zur Bereitstellung eines ständig vorhandenen Magnetfeldes einen Permanentmagneten auf, dessen Magnetfeld durch eine entsprechend angeordnete Magnetspule, an die Spannung angelegt wird, gestärkt oder geschwächt wird.

Alternativ dazu ist vorgesehen, dass das Kraftfeld ein elektrisches Feld und die Hydraulikflüssigkeit eine elektrorheologische Flüssigkeit ist, wobei das Stellglied Elektroden aus einem Elektret aufweist.

Eine besonders kompakte Bauweise des Prothesen-Kniegelenkes und der Dämpfereinrichtung ergibt sich, wenn das Stellglied sich innerhalb eines Kolbens befindet oder in diesen integriert ist, der sich in dem Dämpfungszylinder bewegt, so dass kein zusätzlicher Bauraum benötigt wird, um das Stellglied unterzubringen. In einer Weiterbildung der magnetorheologischen Variante ist vorgesehen, dass der Dämpfungszylinder aus einem ferromagnetischen Material besteht, um einen höheren Integrationsgrad zu erreichen.

In einer Weiterbildung ist vorgesehen, dass zwischen der Zylinderinnenwandung und dem Hydraulikkolben ein Durchgang für den Durchtritt der Hydraulikflüssigkeit vorhanden ist, so dass die Zylinderwandung einen Teil des Magnetkreises oder der Felderregung bildet, der durch den Kolben, den eingelagerten Permanentmagneten oder Elektreten über den Durchgang und den Zylinder geschlossen wird. Vorteilhafterweise ist der Durchgang als ein Ringspalt ausgebildet, so dass eine Leckage um den Kolben herum stattfindet.

Alternativ dazu ist innerhalb des Kolbens ein Durchgang für den Durchtritt der magnetorheologischen oder elektrorheologischen Flüssigkeit ausgebildet, so dass sich der Magnetkreis oder das Feld innerhalb des Kolbens schließt und dort die Viskosität der durchströmenden Flüssigkeit verändert. Bei dieser Ausgestaltung ist es nicht notwendig, dass der Zylinder aus einem ferromagnetischen Material besteht. Der Durchgang kann als Bohrung, Kanal oder Ringspalt mit diamagnetischen Brücken für magnetorheologische Flüssigkeiten ausgebildet sein, wobei auch mehrere Bohrungen oder Kanäle innerhalb des Kolbens oder am Kolbenrand ausgebildet sein können.

Eine vorteilhafte Art der Energiezuleitung zu dem Stellglied besteht in einer Zuleitung durch die Kolbenstange, wodurch sehr stabil und unmittelbar die Energie zu der Magnetspule oder den Elektroden innerhalb des Kolbens geleitet werden kann.

In einer anderen Ausgestaltung der Erfindung ist es vorgesehen, dass die durch den Hydraulikkolben veränderbaren Zylindervolumina durch eine Verbindungs- oder Leckageleitung verbunden sind und dass das Stellglied die magnetorheologische oder elektrorheologische Flüssigkeit in der Leckageleitung bzw. in der Verbindungsleitung mit einem Feld beaufschlagt bzw. in der Leitung das bestehende Feld verändert. Eine solche Anordnung erfordert einen relativ geringen konstruktiven Aufwand und kann mit üblichen Hydraulikmitteln und felderzeugendenen Elementen verwirklicht werden.

Nachfolgend wird anhand von Ausführungsbeispielen die Erfindung näher erläutert. Gleiche Bezugszeichen in verschiedenen Figuren bezeichnen gleiche Baumelemente. Es zeigen:
Figur 1 - eine erste Ausgestaltung einer Dämpereinheit mit einem externen Stellglied;
Figur 2a - eine Dämpfereinheit mit einem in einem Kolben integrierten Stellglied;
Figur 2b - eine Variante der Dämpfereinheit gemäß der Figur 2a; und
Figur 3 - eine Ausbildung der Dämpfereinheit für elektrorheologische Flüssigkeiten.

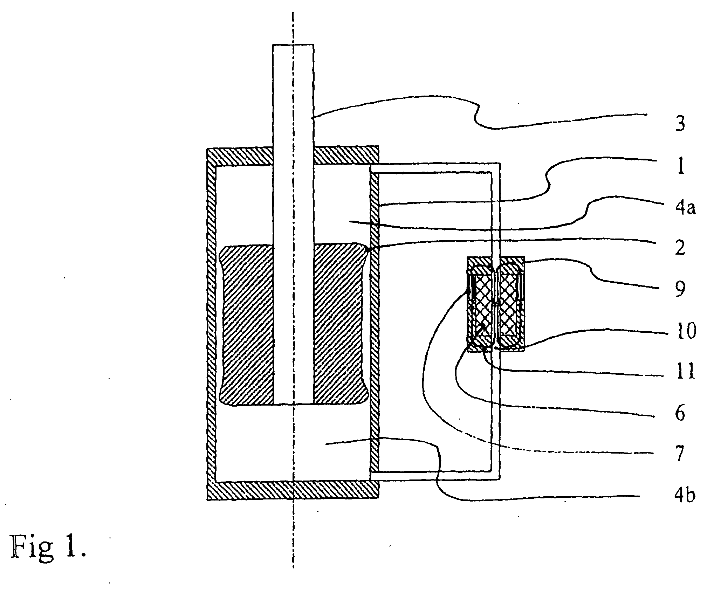

In der Figur 1 ist eine Schnittdarstellung eines hydraulischen Dämpfungszylinders 1 mit einem sich darin bewegenden Hydraulikkolben 2 dargestellt, der über eine Kolbenstange 3 oszillierend innerhalb des Zyllinders 1 bewegt wird. An den Totpunkten des Kolbens 2 ist je ein Anschluß für eine Leckageleitung 10 vorgesehen, die die sich verändernden Zylindervolumina 4a, 4b miteinander verbindet. Bei einer Bewegung des Kolbens 2 innerhalb des Zylinders 1 wird so eine innerhalb des Zylinders 1 befindliche, magnetorheologische Flüssigkeit durch die Leckageleitung 10 gepumpt. Um die Leckageleitung 10 herum ist ein Permanentmagnet 7 angeordnet, innerhalb dessen eine elektronisch steuerbare Magnetspule 6 angeordnet ist, die von Polplatten 9 umschlossen ist, um so bei Anlegen einer Spannung einen geschlossenen Magnetkreis 11 zu realisieren. Der Magnetkreis 11 umschließt die Leckageleitung 10. Je nach Orientierung des durch die Magnetspule 6 erzeugten Magnetfeldes wird das durch den Permanentmagneten 7 gebildete, stets vorhandene Magnetfeld gestärkt oder geschwächt, um so die viskosen Eigenschaften der magnetorheologischen Flüssigkeit entsprechend zu verändern. Durch den Permanentmagneten 7 ist eine Vormagnetisierung bewirkt, mit der eine gewisse Dämpfung voreingestellt ist. Aufgrund dieser Voreinstellung kann ein günstigerer Energieverbrauch aufgrund geringer zusätzlicher Magnetfelder über die Magnetspule 6 erzielt werden.

Somit wird neben einer Voreinstellung auf einen Grunddämpfungswert die Dämpfung elektronisch gesteuert, indem die magnetische Erregung durch die Magnetspule 6 verändert wird, wodurch die magnetorheologische Flüssigkeit, die das Hydraulikmedium darstellt, entsprechend beeinflusst wird.

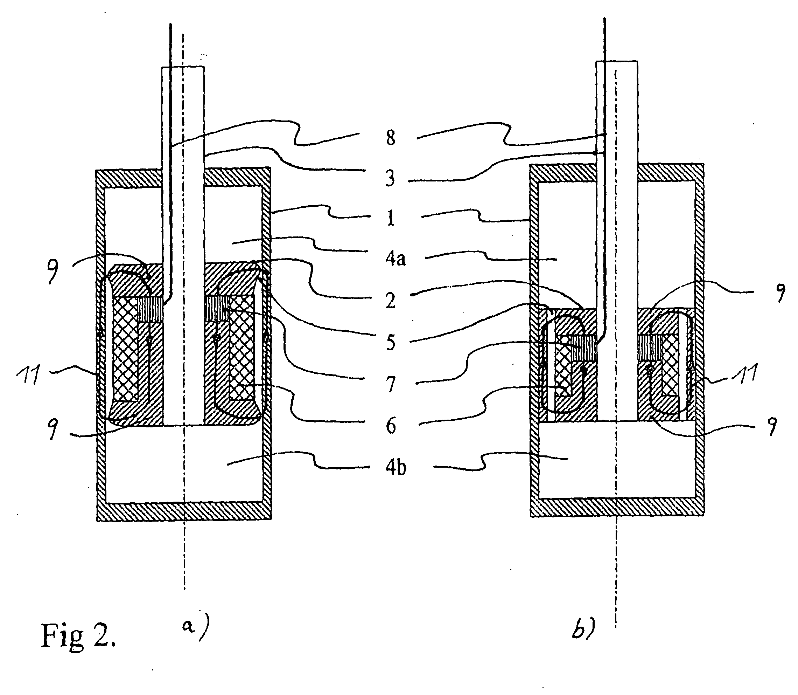

Alternativ dazu ist es vorgesehen, eine geschlossene Ausführungsform der Dämpfereinheit bereitzustellen, bei der die Leckage zwischen den Zylindervolumina 4a, 4b innerhalb des Zylinders 1 auftritt. In der Figur 2a ist eine erste Variante dargestellt, bei der ein Durchgang 5 in Gestalt eines Ringspaltes zwischen dem Kolben 2 und der Zylinderinnenwandung ausgebildet ist, so dass das magnetorheologische Fluid bei einer Bewegung des Kolbens 2 innerhalb des Zylinders 1 durch den Ringspalt 5 hindurch strömt. Innerhalb des Kolbens 2 ist ein Permanentmagnet 7 ausgebildet, der eine Vormagnetisierung bewirkt, die durch den ferromagnetischen Kolben 2, der die Polplatten 9 für die Magnetspule 6 bildet und die ferromagnetische Zylinderwandung bewirkt wird, so dass sich innerhalb dieser Komponenten ein geschlossener Magnetkreis 11 bildet. Durch die über den Permanentmagneten 7 erzielte Vormagnetisierung ist ein gewisses Maß an Dämpfung voreingestellt, die durch Aktivierung der Magnetspule 6 über eine Zuleitung 8, die durch die Kolbenstange 3 geführt wird, verstärkt oder bei einer entsprechend anderen Orientierung verringert wird. Dadurch lassen sich die Dämpfungsgrade des Prothesen-Kniegelenkes über ein weiten Bereich einstellen.

Für den Fall, dass der Zylinder 1 nicht ferromagnetisch ist und somit der Magnetkreis nicht über den ferromangnetischen Kolben 2 mit eingelagerten Permanentmagneten 7 über den Ringspalt 5 und den ferromagnetischen Zylinder 1 geschlossen werden kann, ist in der Figur 2b eine Variante dargestellt, bei dem die Leckage durch den Kolben 2 hindurch geht, wobei der Leckstrom durch einen Durchgang 5, beispielsweise in Gestalt einer Bohrung, eines Kanals oder eines unterbrochenen Ringspaltes ausgebildet ist. Der Kolben 2 schließt mit der Zylinderinnenwandung ab, so dass ein Leckstrom nur durch den innerhalb des Kolbens 2 vorgesehenen Durchgang 5 erfolgt, wobei der Durchgang 5 mit diamagnetischen Brücken versehen ist. In dem Kolben 2 bildet sich somit der Magnetkreis 11 aus, der in der vorliegenden Ausführungsform durch den Permanentmagneten 7 und dem Kolben 2 gebildet wird. Bei Bedarf kann der Magnetkreis 11 bzw. das Magnetfeld durch Aktivierung der Magnetspule 6 verstärkt oder geschwächt werden. Die Zuleitung zu der Magnetspule 6 erfolgt wieder durch die Kolbenstange 3, so dass eine sichere und zuverlässige Ansteuerung der Magnetspule 6 gewährleistet ist.

Der Dämpfungszylinder 1 ist somit ein Hydraulikzylinder, der mit einer gezielten Leckage zwischen den beiden Zylindervolumina 4a, 4b versehen ist, die von dem Hydraulikkolben 2 getrennt werden. Die Leckagerate wird dadurch gesteuert, dass die magnetorheologische Flüssigkeit, die das Hydraulikmedium darstellt, durch ein Magnetfeld beeinflusst wird, das zunächst durch einen Permanentmagneten 7 bereitgestellt wird und somit eine Vormagnetisierung der magnetorheologischen Flüssigkeit und eine Voreinstellung der Dämpfung bewirkt. Bei Bedarf kann durch Anlegen einer Spannung an die Magnetspule 6 die Dämpfung elektronisch gesteuert werden, das heißt, die Viskosität des Hydraulikmediums und dadurch die Dämpfung wird verändert.

Zur Reduzierung der Bauteilanzahl ist vorgesehen, dass die Polplatten 9 von dem Kolben 2 selbst ausgebildet werden, wobei der Kolben 2 aus einem ferromagnetischen Material mit einem eingelagerten oder mehreren eingelagerten Permanentmagneten 7 ausgebildet ist.

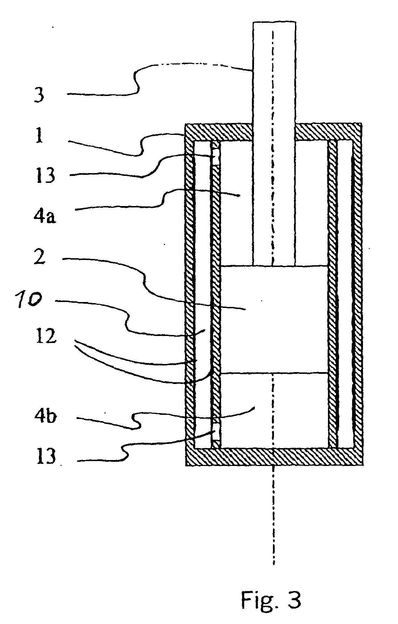

Eine mögliche Ausführungsform des eine Dämpfungszylinders 1 mit einer elektrorheologischen Hydraulikflüssigkeit ist in der Figur 3 dargestellt.

Die Leckage zwischen den Zylindervolumina 4a und 4b erfolgt hier über Mantelbohrungen 13 und eine konzentrisch zum Zylinder 1 angeordnete Leckageleitung 10. Dieser trägt die flächigen Elektroden 12. Werden diese mit einem elektrischen Spannungsfeld beaufschlagt, steigt die Viskosität der eletrorheologischen Flüssigkeit, was den Widerstand gegen die Verschiebung des Kolbens 2 erhöht. Erfindungsgemäß können diese Elektroden 12 als elektrisch vorpolarisiertes Elektret ausgeführt sein.

In allen Ausführungsformen ist der Tatsache nicht Rechnung getragen, dass die Kolbenfläche des oberen Zylindervolumens 4a um die Fläche der Kolbenstange 3 vermindert ist, und somit ein Volumenausgleich oder eine durchgehende Kolbenstange 3 erforderlich wird. Solche Ausgleichsvolumina sind als gas- oder federvorgespannte Lösungen Stand der Technik und werden aus Zwecken der Übersichtlichkeit hier nicht konstruktiv ausgeführt.

## Patentansprüche

1. Prothesen-Kniegelenk mit einem hydraulischen Dämpfungszylinder (1) zur Regulierung der Schwungphasensteuerung und Standphasensicherung, und mit einer elektronischen Steuerung für ein die Hydraulikflüssigkeit des Dämpfungszylinders (1) beaufschlagendes Kraftfeld, wobei die Viskosität der Hydraulikflüssigkeit durch das Kraftfeld veränderbar ist, **gekennzeichnet durch** ein ein permanentes Kraftfeld erzeugendes Stellglied (6, 7, 9, 12), das die Hydraulikflüssigkeit mit einem bestehenden Kraftfeld beaufschlagt, das **durch** das elektronisch gesteuerte Kraftfeld geschwächt oder gestärkt wird.

2. Prothesen-Kniegelenk nach Anspruch 1, **dadurch gekennzeichnet, dass** das Kraftfeld ein Magnetfeld und die Hydraulikflüssigkeit eine magnetorheologische Flüssigkeit ist.

3. Prothesen-Kniegelenk nach Anspruch 1, **dadurch gekennzeichnet, dass** das Stellglied einen Permanentmagneten (7) und eine Magnetspule (6) aufweist.

4. Prothesen-Kniegelenk nach Anspruch 1, **dadurch gekennzeichnet, dass** das Kraftfeld ein elektrisches Feld und die Hydraulikflüssigkeit eine elektrorheologische Flüssigkeit ist.

5. Prothesen-Kniegelenk nach Anspruch 4, **dadurch gekennzeichnet, dass** das Stellglied Elektroden (12) aus einem Elektret aufweist.

6. Prothesen-Kniegelenk nach einem der voranstehenden Ansprüche, **dadurch gekennzeichnet, dass** das Stellglied (6, 7, 9) in einem sich innerhalb des Dämpfungszylinders (1) bewegenden Kolben (2) angeordnet ist.

7. Prothesen-Kniegelenk nach Anspruch 6, **dadurch gekennzeichnet, dass** in dem Kolben (2) oder zwischen der Zylinderinnenwandung und dem Kolben (2) ein Durchgang (5) für den Durchtritt der Hydraulikflüssigkeit ausgebildet ist.

8. Prothesen-Kniegelenk nach Anspruch 6 oder 7, **dadurch gekennzeichnet, dass** der Durchgang (5) als Ringspalt ausgebildet ist.

9. Prothesen-Kniegelenk nach Anspruch 8, **dadurch gekennzeichnet, dass** der Durchgang (5) als Bohrung, Kanal oder Ringspalt mit diamagnetischen Brücken ausgebildet ist.

10. Prothesen-Kniegelenk nach einem der voranstehenden Ansprüche, **dadurch gekennzeichnet, dass** eine Zuleitung (8) zu dem Stellglied (6, 7, 9, 12) durch eine Kolbenstange (3) verläuft.

11. Prothesen-Kniegelenk nach einem der Ansprüche 1 bis 5, **dadurch gekennzeichnet, dass** die durch einen Kolben (2) veränderbaren Zylindervolumina (4a, 4b) durch eine Leckageleitung (10) verbunden sind und das Stellglied (6, 7, 9, 12) die magnetorheologische Flüssigkeit in der Leckageleitung (10) mit einem Magnetfeld oder elektrischen Feld beaufschlagt.

## Claims

1. Knee-joint prosthesis with a hydraulic damping cylinder (1) for regulating the swing phase control and stance phase stabilization, and with an electronic control for a force field acting on the hydraulic fluid of the damping cylinder (1), the viscosity of the hydraulic fluid being able to be changed by the force field, **characterized by** an actuating member (6, 7, 9, 12) which generates a permanent force field and acts on the hydraulic fluid with an existing force field which is weakened or strengthened by the electronically controlled force field.

2. Knee-joint prosthesis according to Claim 1, **characterized in that** the force field is a magnetic field and the hydraulic fluid is a magnetorheological fluid.

3. Knee-joint prosthesis according to Claim 1, **characterized in that** the actuating member has a permanent magnet (7) and a magnet coil (6).

4. Knee-joint prosthesis according to Claim 1, **characterized in that** the force field is an electric field and the hydraulic fluid is an electrorheological fluid.

5. Knee-joint prosthesis according to Claim 4, **characterized in that** the actuating member has electrodes (12) made of an electret.

6. Knee-joint prosthesis according to one of the preceding claims, **characterized in that** the actuating member (6, 7, 9) is arranged in a piston (2) moving inside the damping cylinder (1).

7. Knee-joint prosthesis according to Claim 6, **characterized in that** a passage (5) for the movement of the hydraulic fluid is formed in the piston (2) or between the inside wall of the cylinder and the piston (2).

8. Knee-joint prosthesis according to Claim 6 or 7, **characterized in that** the passage (5) is designed as an annular gap.

9. Knee-joint prosthesis according to Claim 8, **characterized in that** the passage (5) is designed as a bore, channel or annular gap with diamagnetic bridges.

10. Knee-joint prosthesis according to one of the preceding claims, **characterized in that** a feed line (8) to the actuating member (6, 7, 9, 12) extends through a piston rod (3).

11. Knee-joint prosthesis according to one of Claims 1 to 5, **characterized in that** the cylinder volumes (4a, 4b) which can be changed by a piston (2) are connected via a leakage line (10) and the actuating member (6, 7, 9, 12) acts on the magnetorheological fluid in the leakage line (10) with a magnetic field or electric field.

## Revendications

1. Prothèse d'articulation du genou avec cylindre d'amortissement hydraulique (1) pour réguler la commande de la phase de ballant et sécuriser la phase de station debout, et comprenant une commande électronique pour un champ de force agissant sur le fluide hydraulique du cylindre d'amortissement (1), la viscosité du fluide hydraulique étant modifiable par le champ de force, **caractérisée par** un organe de réglage (6, 7, 9, 12) produisant un champ de force permanent, et agissant sur le fluide hydraulique avec un champ de force persistant, qui est renforcé ou diminué par le champ de force commandé électroniquement.

2. Prothèse du genou selon la revendication 1, **caractérisée en ce que** le champ de force est un champ magnétique et le fluide hydraulique est un fluide magnétorhéologique.

3. Prothèse du genou selon la revendication 1, **caractérisée en ce que** l'organe de réglage comporte un aimant permanent (7) et une bobine magnétique (6).

4. Prothèse du genou selon la revendication 1, **caractérisée en ce que** le champ de force est un champ électrique et le fluide hydraulique est un fluide électrorhéologique.

5. Prothèse du genou selon la revendication 4, **caractérisée en ce que** l'organe de réglage comporte des électrodes et un électret.

6. Prothèse du genou selon l'une des revendications précédentes, **caractérisée en ce que** l'organe de réglage (6, 7, 9) est disposé dans un piston (2) se déplaçant à l'intérieur du cylindre d'amortissement (1).

7. Prothèse du genou selon la revendication 6, **caractérisée en ce qu'**un passage (5) destiné à être traversé par le fluide hydraulique est aménagé dans le piston (2) ou entre la paroi intérieure du cylindre et le piston (2).

8. Prothèse du genou selon la revendication 6 ou 7, **caractérisée en ce que** le passage est aménagé sous forme de fente annulaire.

9. Prothèse du genou selon la revendication 8, **caractérisée en ce que** le passage est aménagé sous forme d'alésage, de canal, ou de fente annulaire avec des ponts diamagnétiques.

10. Prothèse du genou selon l'une des revendications précédentes, **caractérisée en ce qu'**une conduite d'alimentation (8) va à l'organe de réglage (6, 7, 9, 12) par une tige de piston (3).

11. Prothèse du genou selon l'une des revendications 1 à 5, **caractérisée en ce que** les volumes du cylindre (4a, 4b) modifiables par un piston (2) sont reliés par une conduite de fuite (10), et **en ce que** l'organe de réglage (6, 7, 9, 12) applique un champ magnétique ou un champ électrique au fluide magnétorhéologique dans la conduite de fuite (10).
